# EUROPEAN PATENT SPECIFICATION

(11) **EP 4 363 874 B1**
(45) Date of publication and mention of the grant of the patent: **27.11.2024**
(21) Application number: 22734316.7
(22) Date of filing: 22.06.2022
(51) Int. Cl.: G01R 33/28, G01R 33/567, A61B 5/00, A61B 5/055, A61B 5/113, A61B 5/16, A61B 5/024, G01S 17/88, A61B 5/0205

(54) **INFRARED CAMERA-BASED PATIENT MONITORING IN MRI**
INFRAROT-KAMERABASIERTE PATIENTENÜBERWACHUNG IN DER MRT
SURVEILLANCE DE PATIENT PAR CAMÉRA INFRAROUGE EN IRM

(30) Priority: 02.07.2021 EP 21183383
(43) Date of publication of application: 08.05.2024
(73) Proprietor: Koninklijke Philips N.V., 5656 AG Eindhoven (NL)
(72) Inventor: WUELBERN, Jan, Hendrik, 5656 AG Eindhoven (NL); LEUSSLER, Christoph, Günther, 5656AG Eindhoven (NL)
(74) Representative: Philips Intellectual Property & Standards
(86) International application number: PCT/EP2022/067032
(87) International publication number: WO 2023/274812

(56) References cited:
- JP-A- 2004 201 977
- SONGXIANG GU ET AL: "Pattern independent deformation estimation illustrated by MRI", NUCLEAR SCIENCE SYMPOSIUM CONFERENCE RECORD, 2008. NSS '08. IEEE (19-25 OCT. 2008), IEEE, PISCATAWAY, NJ, USA, 19 October 2008 (2008-10-19), pages 5285 - 5291, XP031418695, ISBN: 978-1-4244-2714-7
- TREMBLAY M ET AL: "Retrospective Coregistration of Functional Magnetic Resonance Imaging Data Using External Monitoring", MAGNETIC RESONANCE IN MEDICINE, WILEY-LISS, US, vol. 53, no. 1, 1 January 2005 (2005-01-01), pages 141 - 149, XP002619766, ISSN: 0740-3194, [retrieved on 20041222], DOI: 10.1002/MRM.20319

## Description

### FIELD OF THE INVENTION

The present invention relates to the field of magnetic resonance imaging, and, more specifically, the invention relates to a magnetic resonance imaging (MRI) system adapted for camera-based patient observation and/or monitoring of parameters of the patient, such as vital signs and/or movement, based on camera observation and to a method of providing such magnetic resonance imaging system.

### BACKGROUND OF THE INVENTION

Monitoring systems to monitor the examination region of a magnetic resonance examination system with a camera are generally known in the art. For example, a camera for imaging the patient during a magnetic resonance imaging examination may be mounted outside the bore of the MRI system, e.g. on or adjacent to a protective cover of the system. Many variations on and extensions of this concept are known as well. For example, a mirror may be installed in the bore of the MRI system; such that a camera can observe the patient, e.g. the patient's face, even when no direct line of sight is available or would be difficult to achieve. In many configurations the camera system is mounted relatively far away from the region to be observed, and the optical axis of the camera may be, generally, oriented along, or may have a major component in, the direction of the longitudinal axis of the magnet bore. Since a patient is typically, or at least very often, examined with the longitudinal axis of the body substantially aligned with the longitudinal axis of the bore, the use of such mirror can be particularly advantageous in providing a better view to the camera system.

In addition to allowing an operator to observe the patient, e.g. to detect signs of distress, to ensure that the patient remains awake or to assess adherence to an imaging protocol (for example, in functional imaging of the brain), the camera system may also be used in conjunction with an image analysis system to determine useful parameters, for example to detect and/or quantify movement, breathing, heart rate or other cardiac parameters, and/or other vital signs and/or parameters generally indicative of the patient's state. For example, when motion is detected, data that is concomitantly acquired by the MRI system can be labeled to be discarded or to be corrected such as to compensate for the detected motion.

The camera system may comprise an camera that is sensitive in the (human) visual range, but it is also known in the art to use an infrared camera. The use of the infrared range may have several advantages. For example, in JP 2004-041411, a method is disclosed to present a visual stimulus in an MRI system. A dichroic mirror is used to divert an optical path from substantially a front direction onto the eye of the patient, such as to present a visual stimulus from a projector and a screen. Additionally, the eyeball, illuminated by an infrared lamp, is monitored by an infrared camera through the dichroic mirror. Thus, the visual stimulus in the optical (i.e. visual) wavelength spectrum is reflected by the dichroic mirror while infrared light to illuminate and monitor the eye passes through.

SONGXIANG et al, "Pattern independent deformation estimation illustrated by MRI," Nuclear Science Symposium Conference Record, NSS '08, pp. 5285-5291, TREMBLAY et al, "Retrospective coregistration of functional magnetic resonance imaging data using external monitoring," MR in Medicine 53(1), pp. 141-149, and JP 2004 201977, disclose various MRI systems and infared camera systems, in which infrared markers are used to detect positions of the markers using the camera system, e.g. to estimate patient motion and deformation or to aid in aligning the patient with respect to the scanner system.

### SUMMARY OF THE INVENTION

It is an object of embodiments of the present invention to provide in good and efficient means and methods for monitoring a subject in a magnetic resonance imaging system using an infrared camera system, and/or for quantifying vital signs, movement and/or other parameters of interest indicative of the subject based on an output of the infrared camera system.

It is an advantage of embodiments of the present invention that lighting conditions can be improved, e.g. such as to avoid underexposure and/or overexposure of regions in an image provided by the camera, e.g. to provide a more homogeneous illumination of the camera's field of view (or at least a primary region of interest in the field of view).

It is an advantage of embodiments of the present invention that spurious reflections of infrared light, e.g. emitted by one or more infrared light sources to illuminate a scene to be observed by the camera system, can be avoided or reduced, e.g. such that overexposure of parts of the camera image can be avoided. For example, reflections in the field of view of the camera (or outside, if further reflections are involved) off the protective cover of the magnet bore and/or off the walls of the examination room and/or off auxiliary equipment can be reduced and/or avoided.

It is an advantage of embodiments of the present invention that the quality and/or robustness of image processing applied to the output of the camera system can be improved, e.g. due to a better use of the camera's dynamic range and/or due to avoidance (or reduction) of spurious reflections that could confuse the applied algorithm. Thus, for example, vital signs, motion and/or other parameters of interest relating to a state of the subject undergoing the MRI examination can be reliably and accurately detected and/or quantified.

It is also to be noted that such reflections or adverse effects of stray light are not necessarily easily predictable. For example, an algorithm could, in principle, be tuned to compensate for such nuisance factors if the position and effect thereof were to be static and/or predicable. However, when auxiliary equipment is used, such as auxiliary coils, additional patient restraints and/or supporting pillows and the like, these are generally not positioned and/or oriented in a exact, deterministic and reproducible manner. Obviously, also variation in the subject's body and the position/orientation thereof between different examinations could affect the lighting conditions. It should also be considered that such problems with varying lighting conditions could become even more unpredictable when light from a source reflects multiple times before impinging on the camera.

A system and method in accordance with embodiments of the present invention achieves the above objective.

In a first aspect, the present invention relates to a magnetic resonance imaging system in accordance with claim 1, with an examination zone and comprising an infrared camera system for monitoring a subject when undergoing an examination while positioned in the examination zone of the magnetic resonance imaging system. The infrared camera system comprises an infrared camera, i.e. which is sensitive for infrared radiation (even though the spectral range may also extend into the visual range in accordance with some embodiments; embodiments in which the spectral range is substantially limited to the infrared range, or part thereof, are not necessarily excluded in embodiments either). The magnetic resonance imaging system furthermore comprises a coating on at least one wall of a room housing the magnetic resonance imaging system, and/or on an interior wall of a magnet assembly around the examination zone and/or on auxiliary equipment of the magnetic resonance system for use inside the examination zone, e.g. applied to at least one surface of the magnet bore and/or a wall of the examination room and/or auxiliary equipment of the magnetic resonance imaging system, such as an auxiliary coil assembly (e.g. head coil), a patient couch or mattress and/or other auxiliary items for use inside the examination zone. The coating is furthermore adapted to absorb and/or diffuse infrared light so as to avoid spurious reflections of infrared light into a field of view of the infrared camera and/or to provide diffuse auxiliary lighting.

Thus, the infrared camera system may function to oversee the examination zone on the basis of images (whether by single image frames or by dynamic images) acquired by the camera. The camera has a (volumetric) range or field-of-view for which the camera is sensitive to obtain image information from. The coating functions to avoid spurious reflections of infrared light into the field of view of the camera, e.g. by absorbing infrared light at surfaces which are not intended to be observed by the camera, and/or to provide diffuse auxiliary lighting, i.e. an ambient (infrared) lighting. A coating that diffuses reflected light may be particularly advantageous to provide a homogeneous lighting of the examination zone without sharp (unintentional) contrasts, such as shadows or highlights. Particularly, by using such infrared light diffusing coating, a good lighting of the imaged scene may be achieved with few infrared light sources, even one light source, even if this source is point-like (or relatively small). The use of multiple light sources is nevertheless not necessarily excluded.

It is an advantage that non-patient elements (walls, equipment, ...) in the field-of-view of the camera can thus be adapted to avoid excessive stray (IR) light (or more homogeneously diffused light) and allow for an adequate exposure of the patient's region of interest being observed by camera. The wall and/or auxiliary equipment may, for example, be treated by a suitable coating. Additionally, a cloth sheet or blanket with suitable spectral properties (cf. the coating) may be used to cover irrelevant surfaces near the object to be observed, e.g. to cover part of the patient's body.

In a magnetic resonance imaging system in accordance with embodiments of the present invention, the auxiliary equipment may comprise a radiofrequency transmitter/receiver coil and/or a patient couch, and/or other equipment intended for use inside the examination zone while imaging the subject.

The magnetic resonance imaging system in accordance with embodiments of the present invention may comprise a wall of the examination zone (e.g. an interior wall of a housing of a magnet bore of the system), a wall of an examination room (e.g. where the MRI system, or specifically, the magnet bore thereof, is set up) and/or auxiliary equipment, wherein at least a part of said wall and/or auxiliary equipment, e.g. particularly where in the field of view of the camera, may be adapted to absorb (or attenuate) and/or diffusely reflect incident light in the infrared spectrum.

The use of infrared imaging may enable the subject to be monitored under low ambient lighting conditions (in the visible spectrum). For example, it has been observed that at least some patients perceive low lighting or even complete darkness to be more comfortable. Such conditions, however, do not need to interfere with sufficient lighting in the infrared range for camera observation using a system in accordance with embodiments.

In a magnetic resonance imaging system in accordance with embodiments of the present invention, the coating may be adapted for absorbing infrared light, e.g. over a range of operation of the infrared camera system as discussed further hereinbelow, e.g. in a spectral range above 800 nm, e.g. in the range of 800 nm to 900 nm, e.g. in the range of 830 nm to 870 nm, e.g. in a range of 840 nm to 860 nm. For example, the coating may absorb at least 30%, preferably at least 50%, even more preferred at least 70%, e.g. at least 80%, e.g. at least 90%, e.g. at least 95% of the light incident thereon in said infrared range.

In a magnetic resonance imaging system in accordance with embodiments of the present invention, the coating may be adapted for diffusely reflecting infrared light, e.g. over a range of operation of the infrared camera system as discussed further hereinbelow. For example, the coating may reflect at least 30%, preferably at least 50%, even more preferred at least 70%, e.g. at least 80%, e.g. at least 90%, e.g. at least 95% of the light incident thereon in said infrared range. Furthermore, the light may be diffused over a relatively wide angle, e.g. diffusing a unidirectional beam over an angle of at least 10°, e.g. at least 20°, e.g. at least 30°, or even more, e.g. at least 45°, e.g. at least 60° (obviously, the coating not necessarily being adapted for diffusely reflecting only an unidirectional beam).

In a magnetic resonance imaging system in accordance with embodiments of the present invention, the coating may be adapted to reflect and/or transmit at least 50% of incident light in the visible spectrum. For example, the coating may, in addition to its properties in the infrared spectrum range, be adapted to reflect light in the visual spectrum, preferably diffusely to avoid distraction and/or discomfort due to reflections off mirror-like surfaces. The coating may also be substantially transparent in the visible spectrum, such that the color effect (and/or other optical properties) of the underlying material are not (or little) affected by the coating. For example, the coating may reflect or transmit at least 50%, e.g. at least 70%, e.g. at least 85%, e.g. at least 90%, e.g. at least 95%, e.g. at least 99%, of the light in the visible spectrum, e.g. in the range of 350 nm to 800 nm.

In a magnetic resonance imaging system in accordance with embodiments of the present invention, the coating may cover an area, e.g. at least one continuous area, of at least 10 cm², e.g. at least 20 cm², e.g. at least 50 cm², of the (surface of) at least one wall of the room, of the interior wall of the magnet and/or of the auxiliary equipment. For example, the coating may cover substantially the entire area, e.g. at least 50%, e.g. at least 75%, e.g. at least 80%, e.g. at least 90%, of the area (e.g. at least the part thereof within the field of view the camera) of the at least one wall of the room, of the interior wall of the magnet and/or of the (item of, items of and/or all the items of) auxiliary equipment.

In a magnetic resonance imaging system in accordance with embodiments of the present invention, the infrared camera may be adapted to operate in an infrared wavelength range that corresponds to, or substantially overlaps with, the range over which said coating absorbs and/or diffusely reflects infrared light.

In a magnetic resonance imaging system in accordance with embodiments of the present invention, the infrared camera system may comprise an infrared light source configured to direct infrared light into the examination zone.

In a magnetic resonance imaging system in accordance with embodiments of the present invention, the infrared camera (or cameras) may be adapted to operate (e.g. substantially exclusively sensitive to) in a (e.g. narrow) infrared wavelength range and outside the visible wavelength range. This may apply equally to the infrared light source(s), i.e. the infrared light source may emit light exclusively (without limitation thereto) in said infrared wavelength range, or another range that substantially overlaps with the aforementioned wavelength range of the camera. For example, the infrared camera system may operate in an infrared wavelength, e.g. greater than 800 nm, e.g. a wavelength of about 850nm +/- 20nm.

In a magnetic resonance imaging system in accordance with embodiments of the present invention, the infrared camera system may be located outside the examination zone and positioned on or integrated in a flange of the interior wall of the magnet assembly. For example, the light source and the camera may be located outside the examination zone. For a cylindrical bore system, both camera and light source may be located at the flange of the bore enclosure at one end of the bore, which may leave the other end substantially free and may reduce a potential claustrophobic effect on the subject while being imaged by the system.

A magnetic resonance imaging system in accordance with embodiments of the present invention may comprise an image processor to derive information about the subject from the acquired camera image or images. The use of infrared imaging may particularly have advantages for certain applications, and/or may overcome disadvantages of alternatives, e.g. may allow the use of relatively bright illumination without discomfort to the patient and/or personnel. The image processor may be adapted to process image information acquired by the camera system, e.g. to perform static or dynamic image analysis to obtain information from the patient, such as vital signs of the patient, and/or motion of the patient, and/or signs of distress of the patient (or, more generally, patient mood detection). The image processor may be adapted to perform photoplethysmography (PPG) measurements and/or for video-based detection of talking (or recognition of speech, e.g. of simple words or instructions based on facial features). Information on motion of the patient may include, for example, respiratory motion and/or cardiac motion, e.g. indicating the phase of respiratory and/or cardiac cycle phase. For example information on motion of the patient may be derived from image information of the outer hull of the patient's body.

The respiratory and/or cardiac phase information may be applied to a reconstructor of the MRI system (adapted to reconstruct tomographic images from acquired magnetic resonance signals) to correct the acquired magnetic resonance signals for motion or apply motion corrections to the reconstructed magnetic resonance images. For example, a cardiac trigger signal may be determined based on video signals from the camera. Furthermore, other types of motion may also be detected (e.g. quantified) and provided to the reconstructor, such that this information can be taken into account. Examples of other sources of motion, e.g. that are not physiologically induced and/or are less predictable, systematic and/or regular, may include coughing, sneezing, tremors and/or, generally, voluntary motion of the imaged subject.

A magnetic resonance imaging system in accordance with embodiments of the present invention may comprise a reconstructor to reconstruct a magnetic resonance image from magnetic resonance signals acquired by the magnetic resonance system, in which said information determined by the image processor comprises respiratory and/or cardiac phase information and in which the reconstructor is adapted to take this respiratory and/or cardiac phase information into account to correct the acquired signals and/or the reconstructed magnetic resonance image for motion.

A magnetic resonance imaging system in accordance with embodiments of the present invention may comprise a cloth sheet or blanket to cover a part of the body of the subject and/or another object in the examination zone that is not intended to be observed by the infrared camera system, wherein the cloth sheet or blanket is adapted to absorb, attenuate and/or diffusely reflect infrared light.

A magnetic resonance imaging system in accordance with embodiments of the present invention may comprise a mirror, such as a non-metallic mirror, or a reflective surface arranged in the examination zone to reflect infrared light from a body part of the subject, such as the face or part thereof (e.g. the eyes or an eyeball, a region on the forehead, ...), onto the infrared camera and/or to reflect light from the infrared light source onto said body part.

Preferably, the mirror or reflective surface, does not interfere with the radio frequency operation of the magnetic resonance imaging system and/or does not perturb the magnetic fields and the RF dynamic transmission field of the MRI system. Non-metallic mirrors may be particularly suitable for achieving this advantage. For example, the non-metallic mirror may be a dielectric mirror.

In a magnetic resonance imaging system in accordance with embodiments of the present invention, the mirror or reflective surface may be particularly adapted to reflect infrared light, preferably such as to allow the formation of an infrared image of the body part on the imaging plane of the camera. Optionally, the mirror or reflective surface may also be transparent or translucent for light in the (human) visual range.

The mirror or reflective surface can thus arrange for an optical path between a portion of the examination zone and the camera such that the camera can obtain image information from that portion. The mirror(s) or reflective surface(s) may be positioned in the examination zone e.g. by mounting to the inner wall of the magnet bore enclosure. Additionally or alternatively, (the) mirror(s) or reflective surface(s) may also be disposed on auxiliary equipment in the examination zone, such as on a local radio frequency (RF) coil that is positioned on a patient carrier. For example, it may be practical to mount the mirror/reflector onto an RF head coil. At least one mirror or reflective surface may also be provided, alternatively or additionally, in a position outside of the examination zone, e.g. outside the magnet bore.

In a second aspect, the present invention relates to a method in accordance with claim 14 for adapting a magnetic resonance imaging system. The method in accordance with embodiments may be a method for retrofitting a (e.g. preinstalled) magnetic resonance imaging system, or may be integrated in a manufacturing process for manufacturing a magnetic resonance imaging system.

The method comprises obtaining a magnetic resonance imaging system having an examination zone for positioning therein a subject to be imaged by the magnetic resonance imaging system. The magnetic resonance imaging system comprises an infrared camera system, which includes an infrared camera, for monitoring the subject while undergoing such examination.

The method also comprises applying a coating on at least one wall of a room that houses the magnetic resonance imaging system, and/or on an interior wall of a magnet assembly of the system, and/or on auxiliary equipment of the magnetic resonance system for use inside the examination zone, wherein the coating is adapted to absorb and/or diffusely reflect infrared light so as to avoid spurious reflections of infrared light into a field of view of the infrared camera and/or to provide diffuse auxiliary lighting.

Particularly, the coating may be applied to a surface or surfaces which fall within the field of view of the infrared camera (without limitation thereto).

In a method in accordance with embodiments of the present invention, the step of obtaining the magnetic resonance system may comprise modifying a preinstalled magnetic resonance imaging system by installing said infrared camera system (e.g. in addition to applying said coating). Alternatively, the system may be obtained as having the infrared camera system preinstalled.

The independent and dependent claims describe specific and preferred features of the invention. Features of the dependent claims can be combined with features of the independent claims and with features of other dependent claims as deemed appropriate, and not necessarily only as explicitly stated in the claims, provided the resulting combinations fall under the scope of the appended claims.

### BRIEF DESCRIPTION OF THE DRAWINGS

Fig. 1 shows a magnetic resonance imaging system in accordance with embodiments of the present invention.
Fig. 2 shows a photograph of a flange end of a magnet bore of an illustrative magnetic resonance imaging system in accordance with embodiments of the present invention.
Fig. 3 depicts an in-bore camera image as obtainable by a prior-art magnetic resonance imaging system, in which regions of undesirably high exposure due to reflections can be observed.
Fig. 4 illustrates a method in accordance with embodiments of the present invention.

The drawings are schematic and not limiting. Elements in the drawings are not necessarily represented on scale. The present invention is not necessarily limited to the specific embodiments of the present invention as shown in the drawings.

### DETAILED DESCRIPTION OF EMBODIMENTS

Notwithstanding the exemplary embodiments described hereinbelow, is the present invention only limited by the attached claims. The attached claims are hereby explicitly incorporated in this detailed description, in which each claim, and each combination of claims as allowed for by the dependency structure defined by the claims, forms a separate embodiment of the present invention.

The word "comprise," as used in the claims, is not limited to the features, elements or steps as described thereafter, and does not exclude additional features, elements or steps. This therefore specifies the presence of the mentioned features without excluding a further presence or addition of one or more features.

In this detailed description, various specific details are presented. Embodiments of the present invention can be carried out without these specific details. Furthermore, well-known features, elements and/or steps are not necessarily described in detail for the sake of clarity and conciseness of the present disclosure.

In a first aspect, the present invention relates to a magnetic resonance imaging system with an examination zone and comprising an infrared camera system for monitoring a subject when undergoing an examination while positioned in the examination zone.

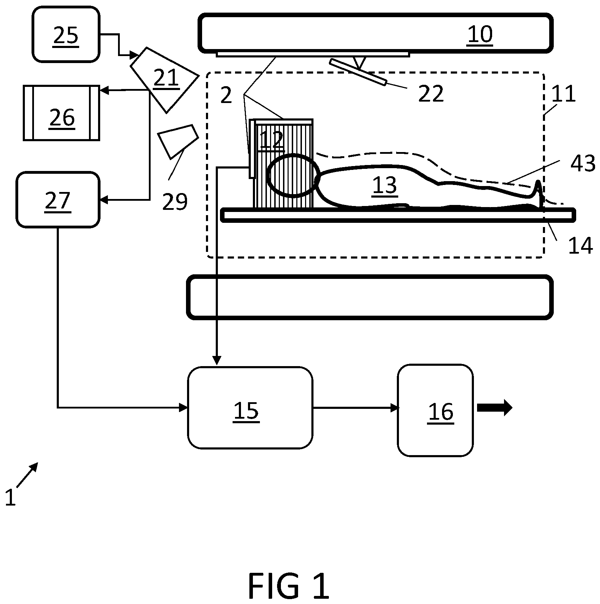

Fig. 1 schematically shows a magnetic resonance imaging system 1 in accordance with embodiments of the present invention. The magnetic resonance examination system comprises a primary magnet assembly 10, which defines the examination zone 11, e.g. the examination zone may be formed by a volume where the magnetic field conditions, as substantially created and controlled by the magnet assembly, are suitable for magnetic resonance imaging. The examination zone may thus correspond to (at least a usable portion of) the volume enclosed by a magnet bore of the system (without limitation, e.g. principles of the present invention equally apply to open bore systems and other, less frequently used, magnet assembly configurations).

A subject, e.g. a patient, to be examined 13 may, in use of the system, be positioned on a patient couch 14 in the examination zone. The primary magnet assembly may comprise magnet windings, e.g. coaxial (e.g. superconductive) windings, to generate a stationary uniform magnetic field in the examination zone. The examination zone may be a cylindrical volume encompassed by these magnet windings.

The system may comprise a reconstructor 15 to reconstruct magnetic resonance image(s), e.g. tomographic MRI images, from magnetic resonance signals acquired by the system in use. The reconstructed images may be provided via an output 16 for viewing, processing or storage.

Auxiliary equipment, such as an RF T/R head coil 12 may, in use, be place in the examination zone to acquire magnetic resonance signals from the subject's head. Other auxiliary coil configurations may be used to acquire signals from other body parts or for different use cases, while, typically, signals may also be received by receiver coils already integrated in the housing of the primary magnet assembly.

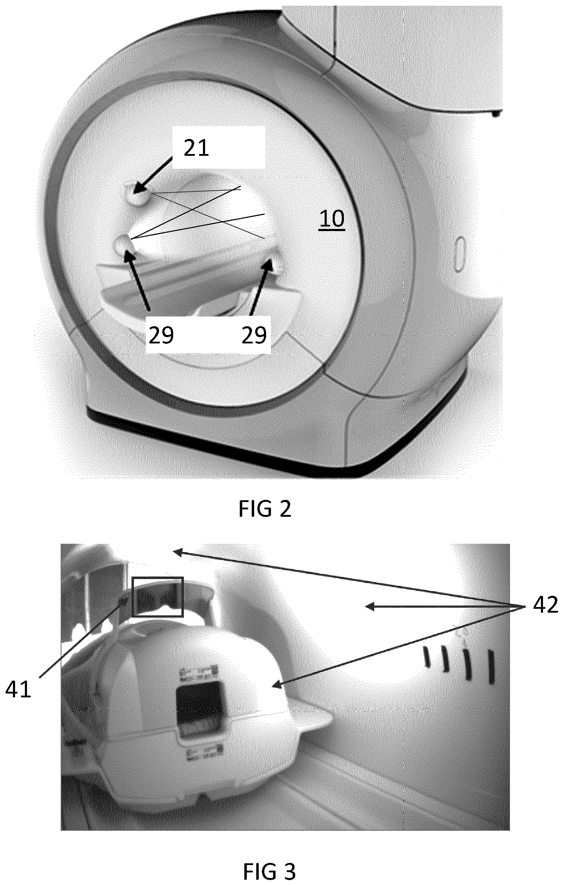

The system comprises an infrared camera system that comprises an infrared camera 21, i.e. which is sensitive for infrared radiation. The infrared camera system is adapted to obtain information from the subject being examined, e.g. to obtain vital signs, motion, indicators of distress and the like. For example, respiratory motion and cardiac motion may be derived from image information of the outer hull of the patient's body. The camera 21 may be mounted close to one entry of the examination zone. For example, the camera may be integrated in, or mounted on, a flange of the MR bore (e.g. such that the usable free bore diameter is not affected or only minimally reduced, and/or to avoid or minimize interference with the operation of the MR system). This is for example illustrated by the photograph in Fig. 2, which also shows the integration of (optional) infrared illuminating lights 29 in this flange.

The camera system may also comprise a light detection and ranging system (LIDAR). This may be useful for generating additional three-dimensional information about the observed scene (e.g. a cloud of 3D localized surface points), which may be taken into account in, for example, image processing of the camera data. For example, a three-dimensional mesh of the (contours of the) patient and/or equipment inside the examination zone may be generated to aid in image processing tasks, such as segmentation, feature recognition, signal extraction and the like. It is to be noted that the coating may be adapted to support such LIDAR data acquisition, or at least to minimize interference with such acquisition. For example, typical LIDAR systems use laser light at 905 nm and/or 1550 nm (without limitation thereto). Thus, the coating may be adapted to avoid reflection in the wavelength(s) in which the LIDAR system operates, or at least to avoid or reduce specular reflection for this wavelength(s). For example, the coating may be adapted to diffusely reflect and/or absorb light for this wavelength(s).

The infrared camera system may also comprise a camera control 25 to control the camera 21, e.g. to adjust parameters such as orientation of the optical axis, focal length, etc.. The infrared camera system may comprise a display 26 to display images of the inside of the examination zone 11 acquired by the camera 21 (raw, or after suitable processing). This enables an operator to visually monitor the subject in the examination zone.

The image information acquired by the camera 21 may also be provided to an image processor 27 (which may for example be implemented in software) to derive information about the subject from the acquired camera image or images.

The image processor 27 may be adapted to process image information acquired by the camera system, e.g. to perform static or dynamic image analysis to obtain information from the patient, such as vital signs of the patient, and/or motion of the patient, and/or signs of distress of the patient (or, more generally, patient mood detection), and/or photoplethysmography (PPG), and/or video-based detection of talking (or recognition of speech, e.g. of simple words or instructions based on facial features). Information on motion of the patient may include, for example, respiratory motion and/or cardiac motion, e.g. indicating the phase of respiratory and/or cardiac cycle phase. For example information on motion of the patient may be derived from image information of the outer hull of the patient's body. The information may be determined by processing (e.g. by an image-based motion detector) and/or by (direct) visual monitoring of the patient via the system by an operator or staff member.

Respiratory and/or cardiac phase information may be provided to the reconstructor 15 to correct the acquired magnetic resonance signals for motion and/or apply motion corrections to the reconstructed magnetic resonance images. For example, a cardiac trigger signal may be determined based on video signals from the camera. Cardiac triggering is particularly useful for cardiac MRI, for obvious reasons, but may also be applied more generally. For example, in neuro-imaging, artefacts in scans of the head and/or neck caused by pulsatile flow of blood and/or cerebrospinal fluid may be suppressed or reduced by such triggering technique or other compensation approach based on a cardiac phase signal. This may also be useful for quantitative measurement of blood flow in the carotid artery. Furthermore, a PPG signal can be extracted from the video signal by analyzing subtle intensity changes of skin pixels, e.g. in the face of a subject, such as at the forehead or at the cheeks. The use of a coating in accordance with embodiments may avoid underexposure and/or overexposure (or at least strong contrasts due to inhomogeneous lighting in the camera's field of view), such that the image processor may produce better, e.g. more accurate and/or more reliable, results, or may be easier to design or configure (since processing to compensate for such inhomogeneous lighting can be reduced or avoided).

The magnetic resonance imaging system furthermore comprises a coating 2 applied to at least one surface of the magnet bore and/or a wall of the examination room and/or auxiliary equipment of the magnetic resonance imaging system, wherein this coating is adapted to absorb and/or diffuse infrared light. The auxiliary equipment may comprise, for example, an RF coil 12, a patient couch 14, or other equipment that may be used inside the examination zone 11. Particularly, the coating may be applied on one or more surfaces which are within a field of view of the infrared camera, as configured to image the subject in use of the system.

Thus, the infrared camera system is adapted to oversee the examination zone on the basis of infrared images. The coating functions to avoid spurious reflections of infrared light into the field of view of the camera, e.g. by absorbing infrared light at surfaces which are not intended to be observed by the camera, and/or to provide diffuse auxiliary lighting, i.e. an ambient (infrared) lighting. A coating that diffuses reflected light may be particularly advantageous to provide a homogeneous lighting of the examination zone without sharp (unintentional) contrasts, such as shadows or highlights. Particularly, by using such light diffusing coating, a good (infrared) lighting of the imaged scene may be achieved with few infrared light sources 29, even one light source, even if this source is point-like (or relatively small). The use of multiple light sources is nevertheless not necessarily excluded, nor the case in which passive IR imaging is used.

A wall of the examination zone and/or auxiliary equipment, such as a (e.g. at least a part of the) wall(s) of the bore (or generally the magnet assembly housing), a (part of the) wall(s) of the examination room, a receive coil(s), and/or any non-patient objects in the examination zone, particularly when in the field of view of the camera, may thus be adapted to absorb and/or diffuse light in the infrared spectrum, e.g. to absorb and/or diffuse light emitted by an infrared light source 29 used for illuminating the camera's field of view. The wall or walls (or parts thereof) may comprise a wall of the examination zone, e.g. an inner wall of the magnet bore enclosure of the MR system, but may (additionally or alternatively) also comprise a wall or walls of the room in which the MR system (e.g. particularly the bore and its associated equipment) is installed, e.g. in cases where the camera 21 is configured to provide a wider overview of examination room (e.g. positioned further removed from the examination zone, as opposed to, for example, being integrated in or on a flange of the bore) and/or where stray light from the walls of the room could significantly influence the image acquired by the camera. The examination room may be intentionally modified and/or constructed to shield the system from external magnetic fields, e.g. may comprise a Faraday cage or similar construct. Thus, it is understood that the examination room, and therefore also its inner walls, may reasonably be considered to be an integral part of the MR imaging system (without necessarily limiting thereto), as opposed to being merely a location where the system is used. Nonetheless, it is noted that it may be advantageous to provide the camera at an edge (with some margin of tolerance) of the examination zone, e.g. at a flange of the bore, such that reflection of light from the walls of the surrounding examination room typically would not significantly influence the image.

It is an advantage that non-patient elements in the field-of-view of the camera can thus be adapted to avoid excessive stray (IR) light and allow for an adequate exposure of the patient's region of interest being observed by camera. This may also, for example, enhance the performance of image processing algorithms, if applied to the camera images, e.g. for motion detection or determination of physiological parameters.

The wall of the examination zone (and/or room) and/or other equipment (including, without limitation, a coil assembly and/or the patient couch) may constitute significant parts of the image collected by the camera. Stray light could therefore cause inadequate exposure of the actual region of interest that is intended to be observed by the camera 21. Poor exposure may lead to a reduced signal to noise ratio, and/or stray light could cause oversaturation of the image. For example, Fig. 3 shows an illustrative camera image (as obtainable by a prior-art system, notably without the coating as described herein), in which a region of interest 41 is underexposed due to excessive stray light 42, which can be overcome by a suitable selection of material properties of the walls and/or outer surfaces of equipment in the field of view and/or a suitable treatment of the visible surfaces, e.g. by a coating, in accordance with embodiments of the present invention.

Additionally, the system may comprise a cloth sheet or blanket 43 with suitable spectral properties (cf. the properties of said coating) to cover irrelevant surfaces near the object to be observed, e.g. to cover part of the patient's body that do not need to be observed by the camera system. Thus, undesirable reflections of infrared light can be further avoided by absorption or diffusion. The discussion hereinbelow regarding spectral properties of the coating may be considered to apply equally to the optical properties of the cloth sheet or blanket. This cloth sheet and/or blanket 43 may be adapted to absorb, attenuate or diffusely reflect infrared radiation, and optionally also to reflect or transmit light in the visible spectrum.

Furthermore, the use of such cloth sheet or blanket may add, advantageously, additional freedom in tuning and/or optimizing the performance of the observation system in use. It will be understood that such blanket can easily be added, repositioned, removed, etc. for a procedure, whereas this is generally not feasible for a fixed coating on the wall and/or auxiliary equipment on a case-by-case basis, except for the likely cost-prohibitive option to provide different versions of (e.g.) a head-coil assembly and/or other equipment items with and without coating, or with different coatings having different optical properties. A cloth sheet or blanket is however highly flexible (figuratively and typically also literally) and configurable.

This may be combined with the use of a quality control system (embodiments of the present invention may include such system, resp. may include a quality control step as part of a method in accordance with embodiments), which may be implemented by dedicated hardware, in software, or a combination of both. For example, the light intensity observed in the acquired camera images (or a calibration image acquired by the camera system) may be evaluated, e.g. checked against a minimum threshold (for example in a region of interest in the image). Other quality criteria may be used as well (alternatively or additionally), such as a minimum and/or maximum image contrast (e.g. in a ROI) and/or a requirement imposed on a signal generated from the camera images, e.g. a signal-to-noise ratio of a signal derived from the acquired images. Thus, an operator may be alerted when the quality control system detects a camera observation setup leading to insufficient image/signal quality, and may be prompted to improve such situation, e.g. by adjusting and/or adding one or more cloth sheets and/or blankets as discussed hereinabove. For example, such online guidance may be provided, e.g. a corresponding software routine may be executed, in a preparation phase for the examination, e.g. when a patient is brought into the scanner bore or shortly thereafter. The quality control system may furthermore be adapted to identify problematic areas in the camera view and/or to generate suggestions on how to improve image quality. As an example, a predictive algorithm may be used, e.g. comprising decision logic tailored by experimentation or modeling for a specific imaging system or system type and/or a trained machine learning algorithm, to evaluate possible interventions (e.g. different blankets and/or different positions/configuration of the blanket) and, on that basis, suggest one or more options that are likely to improve image/signal quality.

In a magnetic resonance imaging system in accordance with embodiments of the present invention, the coating (resp. sheet or blanket) may be adapted for absorbing infrared light, e.g. over a range of operation of the infrared camera system as discussed further hereinbelow, e.g. a spectral range above 800 nm, e.g. in the range of 800 nm to 900 nm, e.g. in the range of 830 nm to 870 nm, e.g. in a range of 840 nm to 860 nm. For example, the coating may absorb at least 30%, preferably at least 50%, even more preferred at least 70%, e.g. at least 80%, e.g. at least 90%, e.g. at least 95%, e.g. in the range of 95% to 100%, of the light incident thereon in said infrared range. The infrared spectral range may preferably correspond, or strongly overlap, with the range of light sensitivity of the infrared camera.

Likewise, the coating may be adapted for diffusely reflecting infrared light, e.g. over a range of operation of the infrared camera system. For example, the coating may reflect at least 30%, preferably at least 50%, even more preferred at least 70%, e.g. at least 80%, e.g. at least 90%, e.g. at least 95% of the light incident thereon in said infrared range. The infrared light may be diffused over a relatively wide angle, e.g. diffusing a unidirectional (incident) beam over an angle (of the reflected light) of at least 10°, e.g. at least 20°, e.g. at least 30°, or even more, e.g. at least 45°, e.g. at least 60° (obviously, the coating not necessarily being adapted for diffusely reflecting only an unidirectional beam, but spreading out and diffusing incident light regardless of the focal properties of the incident wave).

Thus, the magnetic resonance imaging system in accordance with embodiments of the present invention may comprise a wall of the examination zone, a wall of an examination room (e.g. where the MRI system, or specifically, the magnet bore thereof, is set up) and/or auxiliary equipment, wherein at least a part of said wall and/or auxiliary equipment, e.g. particularly where in the field of view of the camera, may be adapted to absorb (or at least attenuate) and/or diffusely reflect incident light in the infrared spectrum.

In a magnetic resonance imaging system in accordance with embodiments of the present invention, the coating may, in addition to its properties in the infrared spectrum range, be adapted to reflect light in the visual spectrum, preferably diffusely to avoid distraction and/or discomfort due to reflections off mirror-like surfaces. For example, this may allow comfortable illumination of the room and MRI system for the subject and good working conditions for personnel. The properties in the visual range may be adapted to provide a predetermined color to the coated surface(s), or may have a broad reflection/diffusion spectral response, e.g. such as to give the impression of a white color (obviously when illuminated accordingly). The coating may absorb a large part of the visual spectrum (e.g. to provide such color of preference to the system), or even substantially the entire visual spectrum. For example, low lighting conditions and/or complete darkness may be more comfortable to at least some subjects, which may be enhanced by absorbing light, by the coating over a large, or substantially the entire, range of the visual spectrum. Nonetheless, this may be less preferred, since low lighting/darkness can alternatively be easily provided by attenuating or extinguishing ambient light sources, whereas a coating in order to reduce the level of ambient lighting (in the visual range) is of a less flexible or even substantially static nature, e.g. cannot easily adapt to subjects who would feel more comfortable in brighter conditions.

However, the coating may be applied as a modification of a premanufactured system (without limitation thereto), in which the material onto which the coating is applied may already have been designed to have predetermined characteristics in the visual range, e.g. to obtain a color and/or brightness of choice for presentation to examinees and workers. Therefore, the coating may also be substantially transparent in the visible spectrum, such that the color effect (and/or other optical properties) of the underlying material are not (or little) affected by the coating. For example, the coating may transmit at least 50%, e.g. at least 70%, e.g. at least 85%, e.g. at least 90%, e.g. at least 95%, e.g. at least 99%, of the light in the visible spectrum, e.g. in the range of 350 nm to 800 nm. In other words, the coating may absorb less than 25%, e.g. less than 10%, of light in the visible spectral range (e.g. the 380 nm to 740 nm range or a substantial part thereof) as perceivable by humans. Such coating may (e.g. alternatively) be reflective to light in the visible spectrum, as discussed hereinabove. This transparent or reflective property has the advantage that the visual appearance of the system is not affected (when directly observed by the eye), e.g. to improve patient comfort (e.g. dark walls or equipment being potentially intimidating, fear-inspiring and/or depressing) and/or for aesthetic considerations.

Suitable coatings are known in the art, for example as used for coating heating elements, where a good absorption of infrared light is advantageous in view of heat transmission and emissivity. Such coatings for heating elements may also be visually transparent or reflective, e.g. particularly when applied to white heating surfaces or heating surfaces of light color. Other suitable coating materials may be selected by the skilled person, e.g. using more sophisticated materials, such as by using metamaterial films to achieve an efficient omnidirectional and broadband optical absorption (or reflection) while simultaneously absorbing or diffusing light in (at least a relevant part of) the infrared spectrum. For the purpose of the present disclosure, surface treatments by applying a paint, a coating, a thin film or a foil are considered equivalent approaches, and are therefore considered to be a 'coating' in the generic sense.

In a magnetic resonance imaging system in accordance with embodiments of the present invention, the infrared camera system may also comprise one or more infrared light sources 29. While embodiments that rely on passive (infrared) lighting for imaging are not necessarily excluded, it will be understood by the skilled person that lighting conditions can be better controlled, and imaging can be more effective, when using active lighting.

The infrared light source 29 may be configured and positioned for directing its light beam directly into/onto the examination zone, possibly being supported by the mirror(s) or reflective surface(s). The use of multiple mirrors may furthermore allow different areas on the subject to be illuminated effectively with a single light source. Embodiments of the present invention furthermore can be particularly suitable for avoiding stray lighting due to undesirable reflections of the infrared light emitted by the infrared source, which reflect the light directly or indirectly (by further reflections) into the observation zone. This can for example be achieved by absorbing the infrared light incident on the coated surface(s) of the system and/or by diffusely reflecting the light by said coating. Even when no active lighting is used, it is noted that heat sources or other sources of infrared radiation in the vicinity of the system may interfere with a passive infrared observation, such that the coating in accordance with embodiments can still be advantageous even in a setting which does not rely explicitly on an infrared light source.

The light source and the camera may be located outside the examination zone, which may simplify the configuration of the magnetic resonance imaging system and may provide for more free bore width in the examination zone. For example, for a cylindrical bore system, both camera and light source may be located at the flange of the bore enclosure at one end of the bore, which may leave the other end substantially free, e.g. to allow unimpeded access to the examination zone (for bringing the patient and/or auxiliary equipment into the examination zone), and reducing a potential claustrophobic effect on the subject, and thus possible discomfort, while being imaged by the system.

The light source(s) 29 may be located next to, or in the vicinity of, the camera, e.g. adjacent to each other at a flange end of the bore or separated by an angle but generally in the same flange region in the longitudinal direction, see e.g. Fig. 2. If the light source and camera are positioned relatively close together, a mirror or reflective surface may be used to bring the emitted infrared light from the source to the examination zone (e.g. a body part to be observed) and back again from the observed examination zone to the camera, e.g. such that the same mirror is used effectively twice in the optical path, thus reducing the number of required mirrors (however, without limitation thereto). Positioning the light source and camera in close proximity to each other may have further advantages, such as minimizing the impact on (reduction of) available bore width and optionally sharing cables or conduits for power supply and/or control signals (which may also simplify design of the system in terms of avoiding or reducing RF and/or magnetic field interference).

In a magnetic resonance imaging system in accordance with embodiments of the present invention, the infrared camera (or cameras) may be adapted to operate (e.g. substantially exclusively sensitive to) in a (e.g. narrow) infrared wavelength range and outside the visible wavelength range. This may apply equally to the infrared light source(s), i.e. the infrared light source may emit light exclusively (without limitation thereto) in said infrared wavelength range, or another range that substantially overlaps with the aforementioned wavelength range of the camera. For example, the infrared camera system may operate in an infrared wavelength, e.g. greater than 800 nm, e.g. a wavelength of about 850nm +/- 20nm.

The use of infrared imaging may have advantages for certain applications, such as detecting blood flow and/or a heart pulse. Furthermore, this may also enable the subject to be monitored under low ambient lighting conditions (in the visible spectrum). For example, it has been observed that at least some patients perceive low lighting or even complete darkness to be more comfortable. Such conditions, however, do not need to interfere with sufficient lighting in the infrared range for camera observation using a system in accordance with embodiments.

The magnetic resonance imaging system may comprise a mirror 22 or reflective surface, such as a non-metallic mirror, arranged in the examination zone, to reflect (infrared) light from a body part of the subject, such as the face or part thereof (e.g. the eyes or an eyeball, a region on the forehead, ...), onto the camera, and/or to reflect light from the light source(s) onto said body part. A (e.g. non-metallic) mirror 22 may be mounted to the inner wall of the examination zone, e.g. of the magnet bore enclosure.

Preferably, the mirror or reflective surface, does not interfere with the radio frequency operation of the magnetic resonance imaging system and/or does not perturb the magnetic fields and the RF dynamic transmission field of the MRI system. Non-metallic mirrors may be particularly suitable for achieving this advantage. For example, the non-metallic mirror may be a dielectric mirror, e.g. comprising a stack of layers of different refractive index, e.g. such that a dielectric resonator is formed by the stack. It is also an advantage that such dielectric mirror comprising a multi-layer stack can be adapted to reflect light in a narrow wavelength range, e.g. the mentioned infrared wavelength range of operation of the infrared camera system, e.g. advantageously even for a relatively large range of angles of incidence.

In a magnetic resonance imaging system in accordance with embodiments of the present invention, the mirror or reflective surface may be particularly adapted to reflect infrared light, preferably such as to allow the formation of an infrared image of the body part on the imaging plane of the camera. Optionally, the mirror or reflective surface may also be transparent or translucent for light in the (human) visual range.

The mirror or reflective surface may thus arrange an optical pathway between a portion of the examination zone, i.e. where the body part is located when the subject is undergoing examination, and the camera.

The mirror or reflective surface can thus arrange for an optical path between a portion of the examination zone and the camera such that the camera can obtain image information from that portion. The mirror(s) or reflective surface(s) may be positioned in the examination zone e.g. by mounting to the inner wall of the magnet bore enclosure. Additionally or alternatively, (the) mirror(s) or reflective surface(s) may also be disposed on auxiliary equipment in the examination zone, such as on a local radio frequency (RF) coil that is positioned on a patient carrier. For example, it may be practical to mount the mirror/reflector onto an RF head coil. At least one mirror or reflective surface may also be provided, alternatively or additionally, in a position outside of the examination zone, e.g. outside the magnet bore.

The use of (a) mirror/reflector(s) may be particularly advantageous when the region of interest for observation is blocked from direct view by the camera. For example, an obstacle may be formed by an opaque object, e.g. auxiliary equipment such as local radiofrequency (RF) transmit or receive (T/R) antennas, located in the camera's direct line of sight into the examination zone. Other possible obstacles include parts of the subject's body.

The mirror or mirrors may also be adapted to allow for repositioning and/or reorienting to take changing (camera) imaging conditions into account, e.g. by allowing the mirror to be attached to a support (e.g. a bore wall) at different positions, by using a sliding rail arrangement, by using a swivel mount onto which the mirror is mounted, and/or other such means. In other words, the mirror or reflective surface may be mounted in an adjustable manner, e.g. by a hinge or pivot for adjustment of its orientation and/or by a sliding rail, movable mount or removable attachment (particularly attachable at multiple possible mounting points) for adjustment of its position. This enables to vary the orientation and/or position of the mirror, e.g. of the orientation relative to the longitudinal axis of the examination zone and/or position along this axis (not excluding adjustments relative to other axes). This provides the flexibility of circumventing obstacles in the examination zone, which may vary between examinations (or even within). Alternatively, the mirror may be mounted to the inner wall, e.g. in a fixed arrangement, so that only little space in the examination zone is taken up by the mirror.

Alternatively, (or additionally) the mirror may be mounted on, or formed as part of, a head T/R coil, e.g. as used for cervical, cranial and/or neuroradiological MR examinations. It is to be noted that integrating the mirror in or on the head coil may avoid costly or complex modification of existing equipment, e.g. of the scanner bore. While a relatively far distance between the camera, e.g. mounted on a flange of the bore, may result in a very limited field of view, e.g. only showing the forehead or part thereof, this may be sufficient for some applications, e.g. to monitor blood pulsation by slight variations in pixel intensity.

In a second aspect, the present invention relates to a method for adapting a magnetic resonance imaging system. The method in accordance with embodiments may be a method for retrofitting a (e.g. preinstalled) magnetic resonance imaging system, or may be integrated in a manufacturing process for manufacturing a magnetic resonance imaging system.

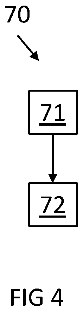

Fig. 4 shows an illustrative method 70 in accordance with embodiments of the present invention.

The method comprises obtaining 71 a magnetic resonance imaging system having an examination zone for positioning a subject in for examination by magnetic resonance imaging. For example, the examination zone may be formed by the volume enclosed by the bore of a magnet assembly of the system, or by a substantial (e.g. usable for imaging) portion thereof.

The magnetic resonance imaging system comprises an infrared camera system, comprising an infrared camera, for monitoring a subject when undergoing an examination while positioned in the examination zone. The step of obtaining 71 the magnetic resonance system may comprise modifying a preinstalled magnetic resonance imaging system by installing the infrared camera system, or the infrared camera system may already be preinstalled in the system. The infrared camera system, as preinstalled or as installed as part of the method 70, may comprise one or more infrared light sources configured to direct infrared light into the examination zone. For example, the infrared camera system may be configured to direct light from the source into the examination zone, e.g. onto a subject in use of the system, such that a thus illuminated part of the body of the subject, positioned within the field of view of the infrared camera, can be imaged by infrared camera. It is noted that optionally a mirror(s) or reflector(s) can be used to guide the infrared light from source to body part to be imaged and/to from the body part to be imaged to the camera. It is also noted that such mirror or reflector can be provided as configurable means, e.g. having adjustable position and/or orientation, i.e. when the system is used to prepare an imaging session for a subject.

The method 70 comprises applying 72 a coating on at least one wall of a room that forms a housing of the magnetic resonance imaging system, and/or on an interior wall of a magnet assembly of the system, and/or on auxiliary equipment of the magnetic resonance system for use inside the examination zone. This coating is furthermore adapted to absorb and/or diffusely reflect infrared light. Such auxiliary equipment may for example comprise a radiofrequency transmitter/receiver coil and/or a patient couch. For the purposes of the present disclosure, 'coating' should not be narrowly construed, and may also refer to a paint, a film, a foil or other suitable surface treatment. The coating may also be multilayered, e.g. comprise multiple stacked coatings, paints, films and/or foils, or combinations thereof.

Particularly, the coating may be applied on one or more surfaces which are within a field of view of the infrared camera, as configured to image the subject in use of the system. The coating may be adapted for absorbing infrared light in a spectral range above 800 nm by at least 30%. The coating may be adapted for diffusely reflecting at least 30% of incident infrared light in a spectral range above 800 nm.

In a method in accordance with embodiments of the present invention, the coating may furthermore be adapted to reflect and/or transmit at least 50% of incident light in the visible spectrum. The spectral properties of the coating may be tuned to the wavelength range of operation of the infrared camera. For example, the infrared camera may be adapted to operate in an infrared wavelength range that corresponds to, or substantially overlaps with, the range over which the coating absorbs and/or diffusely reflects infrared light. In a specific example, the infrared camera system may be adapted to operate in an infrared wavelength of about 850nm +/- 20nm, and the coating may be adapted to substantially absorb and/or reflect infrared light in at least this range.

An image processor may be provided, as part of the method, or may already be comprised in the MRI system as obtained. Such image processor may generally be adapted to derive information about the subject from a camera image or images acquired by the infrared camera system.

The method may also comprise providing a cloth sheet or blanket to cover a part of the body of the subject and/or another object in the examination zone, in use of the system as modified by the method in accordance with embodiments, in which this cloth sheet or blanket is adapted to absorb, attenuate and/or diffusely reflect infrared light, e.g. generally having similar (optical/spectral) characteristics as the coating mentioned hereinabove. Thus, the magnetic resonance system as provided by the method may be considered as a kit of parts, in accordance with some embodiments, comprising the magnetic resonance imaging system with an infrared camera system, wherein the MRI system is modified by coating surfaces of the MRI system as described hereinabove, in combination with the cloth sheet or blanket. The infrared absorption and/or diffuse reflection provided by the coating and/or blanket may thus, in use, enable operators to obtain good infrared imaging quality (and/or robust data inferred therefrom),

Other features, or details of the features described hereinabove, of a method in accordance with embodiments of the second aspect of the present invention shall be clear in view of the description provided hereinabove relating to a system in accordance with embodiments of the first aspect of the present invention.

## Claims

1. A magnetic resonance imaging system (1), the magnetic resonance imaging system comprising:
- an infrared camera system for monitoring a subject when undergoing an examination while positioned in an examination zone (11) of the magnetic resonance imaging system, the infrared camera system comprising an infrared camera (21), and
- a coating (2) on at least one wall of a room housing the magnetic resonance imaging system, and/or on an interior wall of a magnet assembly (10) around the examination zone and/or on auxiliary equipment of the magnetic resonance system for use inside the examination zone,
wherein said infrared camera (21) is adapted to operate in an infrared wavelength range that corresponds to, or substantially overlaps with, the range over which said coating (2) absorbs and/or diffusely reflects infrared light, **characterized in that** said coating is adapted to absorb and/or diffusely reflect infrared light, so as to avoid spurious reflections of infrared light into a field of view of the infrared camera and/or to provide diffuse auxiliary lighting.

2. The magnetic resonance imaging system of claim 1, wherein said auxiliary equipment comprises a radiofrequency transmitter/receiver coil (12) and/or a patient couch (14).

3. The magnetic resonance imaging system of any of the previous claims, wherein said coating (2) is adapted for absorbing infrared light in a spectral range above 800 nm by at least 30%.

4. The magnetic resonance imaging system of any of the previous claims, wherein said coating (2) is adapted for diffusely reflecting at least 30% of incident infrared light in a spectral range above 800 nm.

5. The magnetic resonance imaging system of any of the previous claims, wherein said coating (2) is adapted to reflect and/or transmit at least 50% of incident light in the visible spectrum.

6. The magnetic resonance imaging system of any of the previous claims, wherein said coating covers an area of at least 10 cm² of the surface of said at least one wall of the room and/or said interior wall and/or said auxiliary equipment.

7. The magnetic resonance imaging system of any of the previous claims, wherein said infrared camera system comprises an infrared light source (29) configured to direct infrared light into the examination zone.

8. The magnetic resonance imaging system of any of the previous claims, wherein said infrared camera system is located outside the examination zone and positioned on or integrated in a flange of the interior wall of the magnet assembly.

9. The magnetic resonance imaging system of any of the previous claims, comprising an image processor (27) to derive information about the subject from the acquired camera image or images.

10. The magnetic resonance imaging system of claim 9, wherein said information comprises a value indicative of vital signs, motion and/or mood of the subject.

11. The magnetic resonance imaging system of claim 9 or claim 10, comprising a reconstructor (15) to reconstruct a magnetic resonance image from magnetic resonance signals acquired by the magnetic resonance system, wherein said information determined by said image processor (27) comprises respiratory and/or cardiac phase information and wherein said reconstructor is adapted to take said respiratory and/or cardiac phase information into account to correct said acquired signals and/or said reconstructed magnetic resonance image for motion.

12. The magnetic resonance imaging system of any of the previous claims, comprising a cloth sheet or blanket (43) to cover a part of the body of the subject and/or another object in the examination zone that is not intended to be observed by the infrared camera system, wherein said cloth sheet or blanket is adapted to absorb, attenuate and/or diffusely reflect infrared light.

13. The magnetic resonance imaging system of any of the previous claims, comprising at least one mirror (22) or reflective surface arranged in the examination zone to reflect infrared light from a body part of the subject onto the infrared camera and/or to reflect light from the infrared light source onto said body part.

14. A method (70) for adapting a magnetic resonance imaging system, the method comprises:
- obtaining (71) a magnetic resonance imaging system having an examination zone for positioning therein a subject to be imaged by the magnetic resonance imaging system, in which the magnetic resonance imaging system comprises an infrared camera system, which includes an infrared camera, for monitoring the subject while undergoing examination, and
- applying (72) a coating on at least one wall of a room that houses the magnetic resonance imaging system, and/or on an interior wall of a magnet assembly of the system, and/or on auxiliary equipment of the magnetic resonance system for use inside the examination zone,
wherein said infrared camera is adapted to operate in an infrared wavelength range that corresponds to, or substantially overlaps with, the range over which said coating absorbs and/or diffusely reflects infrared light, **characterized in that** said coating is adapted to absorb and/or diffusely reflect infrared light so as to avoid spurious reflections of infrared light into a field of view of the infrared camera and/or to provide diffuse auxiliary lighting.

15. The method of claim 14, wherein said step of obtaining (71) the magnetic resonance system comprises modifying a preinstalled magnetic resonance imaging system by installing said infrared camera system.

## Patentansprüche

1. Magnetresonanz-Bildgebungssystem (1), wobei das Magnetresonanz-Bildgebungssystem umfasst:
- ein Infrarotkamerasystem zum Überwachen eines Subjekts, wenn es sich einer Untersuchung unterzieht, während es in einem Untersuchungsbereich (11) des Magnetresonanz-Bildgebungssystems positioniert ist, wobei das Infrarotkamerasystem eine Infrarotkamera (21) umfasst und
- eine Beschichtung (2) an mindestens einer Wand eines Raums, in dem sich das Magnetresonanz-Bildgebungssystem befindet, und/oder an einer Innenwand einer Magnetanordnung (10) um den Untersuchungsbereich und/oder an einer Hilfsausrüstung des Magnetresonanzsystems zur Verwendung innerhalb des Untersuchungsbereichs, wobei die Infrarotkamera (21) angepasst ist, in einem Infrarot-Wellenlängenbereich zu arbeiten, der dem Bereich entspricht oder sich im Wesentlichen damit überschneidet, über den die Beschichtung (2) Infrarotlicht absorbiert und/oder diffus reflektiert, **dadurch gekennzeichnet, dass** die Beschichtung angepasst ist, Infrarotlicht zu absorbieren und/oder diffus zu reflektieren, um unechte Reflexionen von Infrarotlicht in ein Sichtfeld der Infrarotkamera zu vermeiden und/oder um eine diffuse Hilfsbeleuchtung bereitzustellen.

2. Magnetresonanz-Bildgebungssystem nach Anspruch 1, wobei die Hilfsausrüstung eine Hochfrequenz-Sender-/Empfängerspule (12) und/oder eine Patientenliege (14) umfasst.

3. Magnetresonanz-Bildgebungssystem nach einem der vorstehenden Ansprüche, wobei die Beschichtung (2) zum Absorbieren von mindestens 30% Infrarotlicht in einem Spektralbereich über 800 nm angepasst ist.

4. Magnetresonanz-Bildgebungssystem nach einem der vorstehenden Ansprüche, wobei die Beschichtung (2) zum diffusen Reflektieren von mindestens 30 % an einfallendem Infrarotlicht in einem Spektralbereich über 800 nm angepasst ist.

5. Magnetresonanz-Bildgebungssystem nach einem der vorstehenden Ansprüche, wobei die Beschichtung (2) angepasst ist, mindestens 50% an einfallendem Licht im sichtbaren Spektrum zu reflektieren und/oder durchzulassen.

6. Magnetresonanz-Bildgebungssystem nach einem der vorstehenden Ansprüche, wobei die Beschichtung eine Fläche von mindestens 10 cm² der Oberfläche der mindestens einen Wand des Raums und/oder der Innenwand und/oder der Hilfsausrüstung abdeckt.

7. Magnetresonanz-Bildgebungssystem nach einem der vorstehenden Ansprüche, wobei das Infrarotkamerasystem eine Infrarotlichtquelle (29) umfasst, die konfiguriert ist, um Infrarotlicht in den Untersuchungsbereich zu richten.

8. Magnetresonanz-Bildgebungssystem nach einem der vorstehenden Ansprüche, wobei sich das Infrarotkamerasystem außerhalb des Untersuchungsbereichs befindet und auf einem Flansch der Innenwand der Magnetanordnung positioniert oder darin integriert ist.

9. Magnetresonanz-Bildgebungssystem nach einem der vorstehenden Ansprüche, das einen Bildprozessor (27) umfasst, um Informationen über das Subjekt aus dem oder den erfassten Kamerabild(ern) abzuleiten.

10. Magnetresonanz-Bildgebungssystem nach Anspruch 9, wobei die Informationen einen Wert umfassen, der auf Lebenszeichen, Bewegung und/oder Stimmung des Subjekts hinweist.

11. Magnetresonanz-Bildgebungssystem nach Anspruch 9 oder Anspruch 10, das einen Rekonstruktor (15) umfasst, um ein Magnetresonanzbild aus Magnetresonanzsignalen zu rekonstruieren, die vom Magnetresonanzsystem erfasst werden, wobei die von dem Bildprozessor (27) bestimmten Informationen Atmungs- und/oder Herzphaseninformationen umfassen, und wobei der Rekonstruktor angepasst ist, um die Atmungs-- und/oder Herzphaseninformationen zu berücksichtigen, um die erfassten Signale und/oder das rekonstruierte Magnetresonanzbild für Bewegung zu korrigieren.

12. Magnetresonanz-Bildgebungssystem nach einem der vorstehenden Ansprüche, das ein Tuch oder eine Decke (43) umfasst, um einen Teil des Körpers des Subjekts und/oder ein anderes Objekt im Untersuchungsbereich, das nicht dazu bestimmt ist, von dem Infrarotkamerasystem beobachtet zu werden, zu bedecken, wobei das Tuch oder die Decke angepasst ist, um Infrarotlicht zu absorbieren, zu dämpfen und/oder diffus zu reflektieren.

13. Magnetresonanz-Bildgebungssystem nach einem der vorstehenden Ansprüche, das mindestens einen Spiegel (22) oder eine reflektierende Oberfläche umfasst, der/die im Untersuchungsbereich angeordnet ist, um Infrarotlicht von einem Körperteil des Subjekts auf die Infrarotkamera zu reflektieren und/oder um Licht von der Infrarotlichtquelle auf das Körperteil zu reflektieren.

14. Verfahren (70) zum Anpassen eines Magnetresonanz-Bildgebungssystems, wobei das Verfahren umfasst:
- Erhalten (71) eines Magnetresonanz-Bildgebungssystems, das einen Untersuchungsbereich zum Positionieren eines Subjekts darin, um durch das Magnetresonanz-Bildgebungssystem abgebildet zu werden, aufweist, wobei das Magnetresonanz-Bildgebungssystem ein Infrarotkamerasystem umfasst, das eine Infrarotkamera zum Überwachen des Subjekts während es sich einer Untersuchung unterzieht, beinhaltet, und
- Aufbringen (72) einer Beschichtung an mindestens einer Wand eines Raums, in dem sich das Magnetresonanz-Bildgebungssystem befindet, und/oder einer Innenwand einer Magnetanordnung des Systems, und/oder einer Hilfsausrüstung des Magnetresonanzsystems zur Verwendung innerhalb des Untersuchungsbereichs,
wobei die Infrarotkamera angepasst ist, in einem Infrarot-Wellenlängenbereich zu arbeiten, der dem Bereich entspricht oder sich im Wesentlichen damit überschneidet, über den die Beschichtung Infrarotlicht absorbiert und/oder diffus reflektiert, **dadurch gekennzeichnet, dass** die Beschichtung angepasst ist, Infrarotlicht zu absorbieren und/oder diffus zu reflektieren, um unechte Reflexionen von Infrarotlicht in ein Sichtfeld der Infrarotkamera zu vermeiden und/oder um eine diffuse Hilfsbeleuchtung bereitzustellen.

15. Verfahren nach Anspruch 14, wobei der Schritt zum Erhalten (71) des Magnetresonanzsystems Modifizieren eines vorinstallierten Magnetresonanz-Bildgebungssystems durch Installieren des Infrarotkamerasystems umfasst.

## Revendications

1. Système d'imagerie par résonance magnétique (1), le système d'imagerie par résonance magnétique comprenant :
- un système de caméra infrarouge pour surveiller un sujet lorsqu'il subit un examen tout en étant positionné dans une zone d'examen (11) du système d'imagerie par résonance magnétique, le système de caméra infrarouge comprenant une caméra infrarouge (21), et
- un revêtement (2) sur au moins une paroi d'une pièce abritant le système d'imagerie par résonance magnétique, et/ou sur une paroi intérieure d'un ensemble magnétique (10) autour de la zone d'examen et/ou sur un équipement auxiliaire du système de résonance magnétique destiné à être utilisé à l'intérieur de la zone d'examen, dans lequel ladite caméra infrarouge (21) est adaptée pour fonctionner dans une plage de longueurs d'onde infrarouge qui correspond à, ou chevauche sensiblement, la plage sur laquelle ledit revêtement (2) absorbe et/ou réfléchit de manière diffuse la lumière infrarouge, **caractérisé en ce que** ledit revêtement est adapté pour absorber et/ou réfléchir de manière diffuse la lumière infrarouge, afin d'éviter les réflexions parasites de la lumière infrarouge dans le champ de vision de la caméra infrarouge et/ou pour fournir un éclairage auxiliaire diffus.

2. Système d'imagerie par résonance magnétique selon la revendication 1, dans lequel ledit équipement auxiliaire comprend une bobine émettrice-réceptrice de radiofréquences (12) et/ou une table de patient (14).

3. Système d'imagerie par résonance magnétique selon l'une quelconque des revendications précédentes, dans lequel ledit revêtement (2) est adapté pour absorber la lumière infrarouge dans une plage spectrale supérieure à 800 nm d'au moins 30 %.

4. Système d'imagerie par résonance magnétique selon l'une quelconque des revendications précédentes, dans lequel ledit revêtement (2) est adapté pour réfléchir de manière diffuse au moins 30 % de la lumière infrarouge incidente dans une plage spectrale supérieure à 800 nm.

5. Système d'imagerie par résonance magnétique selon l'une quelconque des revendications précédentes, dans lequel ledit revêtement (2) est adapté pour réfléchir et/ou transmettre au moins 50 % de la lumière incidente dans le spectre visible.

6. Système d'imagerie par résonance magnétique selon l'une quelconque des revendications précédentes, dans lequel ledit revêtement couvre une aire d'au moins 10 cm² de la surface de ladite au moins une paroi de la pièce et/ou ladite paroi intérieure et/ou ledit équipement auxiliaire.

7. Système d'imagerie par résonance magnétique selon l'une quelconque des revendications précédentes, dans lequel ledit système de caméra infrarouge comprend une source de lumière infrarouge (29) configurée pour diriger la lumière infrarouge dans la zone d'examen.

8. Système d'imagerie par résonance magnétique selon l'une quelconque des revendications précédentes, dans lequel ledit système de caméra infrarouge est situé à l'extérieur de la zone d'examen et positionné sur ou intégré dans une bride de la paroi intérieure de l'ensemble magnétique.

9. Système d'imagerie par résonance magnétique selon l'une quelconque des revendications précédentes, comprenant un processeur d'image (27) pour déduire des informations sur le sujet à partir de l'image ou des images de caméra acquises.

10. Système d'imagerie par résonance magnétique selon la revendication 9, dans lequel lesdites informations comprennent une valeur indicative de signes vitaux, de mouvement et/ou d'humeur du sujet.

11. Système d'imagerie par résonance magnétique selon la revendication 9 ou la revendication 10, comprenant un reconstructeur (15) pour reconstruire une image de résonance magnétique à partir de signaux de résonance magnétique acquis par le système de résonance magnétique, dans lequel lesdites informations déterminées par ledit processeur d'image (27) comprennent des informations respiratoires et/ou de phase cardiaque et dans lequel ledit reconstructeur est adapté pour prendre lesdites informations respiratoires et/ou de phase cardiaque en compte pour corriger lesdits signaux acquis et/ou ladite image de résonance magnétique reconstruite pour le mouvement.

12. Système d'imagerie par résonance magnétique selon l'une quelconque des revendications précédentes, comprenant une couverture ou un drap en tissu (43) pour recouvrir une partie du corps du sujet et/ou un autre objet dans la zone d'examen qui n'est pas destiné à être observé par le système de caméra infrarouge, dans lequel ladite couverture ou ledit drap en tissu est adapté pour absorber, atténuer et/ou réfléchir de manière diffuse la lumière infrarouge.

13. Système d'imagerie par résonance magnétique selon l'une quelconque des revendications précédentes, comprenant au moins un miroir (22) ou une surface réfléchissante agencé dans la zone d'examen pour réfléchir la lumière infrarouge d'une partie du corps du sujet sur la caméra infrarouge et/ou pour réfléchir la lumière de la source de lumière infrarouge sur ladite partie du corps.

14. Procédé (70) d'adaptation d'un système d'imagerie par résonance magnétique, le procédé comprenant :
- l'obtention (71) d'un système d'imagerie par résonance magnétique présentant une zone d'examen pour y positionner un sujet à imager par le système d'imagerie par résonance magnétique, dans lequel le système d'imagerie par résonance magnétique comprend un système de caméra infrarouge, qui inclut une caméra infrarouge, pour surveiller le sujet pendant qu'il subit un examen, et
- l'application (72) d'un revêtement sur au moins une paroi d'une pièce abritant le système d'imagerie par résonance magnétique, et/ou sur une paroi intérieure d'un ensemble magnétique du système, et/ou sur un équipement auxiliaire du système de résonance magnétique pour utilisation à l'intérieur de la zone d'examen,
dans lequel ladite caméra infrarouge est adaptée pour fonctionner dans une plage de longueurs d'onde infrarouge qui correspond à, ou chevauche sensiblement, la plage sur laquelle ledit revêtement absorbe et/ou réfléchit de manière diffuse la lumière infrarouge, **caractérisé en ce que** ledit revêtement est adapté pour absorber et/ou réfléchir de manière diffuse la lumière infrarouge afin d'éviter les réflexions parasites de la lumière infrarouge dans le champ de vision de la caméra infrarouge et/ou pour fournir un éclairage auxiliaire diffus.

15. Procédé selon la revendication 14, dans lequel ladite étape d'obtention (71) du système de résonance magnétique comprend la modification d'un système d'imagerie par résonance magnétique pré-installé en installant ledit système de caméra infrarouge.
